# EUROPÄISCHE PATENTANMELDUNG

(11) **EP 1 516 595 A1**
(43) Veröffentlichungstag der Anmeldung: **23.03.2005**
(21) Anmeldenummer: 03021248.4
(22) Anmeldetag: 18.09.2003
(51) Int. Cl.: A61C 19/05, G01L 5/00

(54) **Vorrichtung zur Bestimmung des Drucks zwischen zwei aneinander gepressten Körpern und dessen Verwendung**

(71) Anmelder: Starek, Bruno, 5201 Seekirchen (AT); Vekörrer, Franz, 5020 Salzburg (AT); Purner, Helmut, 5203 Köstendorf (AT)
(72) Erfinder: Starek, Bruno, 5201 Seekirchen (AT); Vekörrer, Franz, 5020 Salzburg (AT); Purner, Helmut, 5203 Köstendorf (AT)
(74) Vertreter: Berngruber, Otto, Dr.

(57) **Zusammenfassung**

Zur Bestimmung des Drucks zwischen zwei aneinander gepressten Körpern, insbesondere des Dentalkontaktdrucks, ist ein Oszillator (1) mit einer Induktionsspule (2) und ein elektrisch leitfähiger Sensor (11) vorgesehen. Der Sensor (11) ist um die Induktionsspule (2) des Oszillators (1) angeordnet. Bei Änderung der Induktivität der Induktionsspule (2) durch Druckeinwirkung auf den Sensor (11) ändert sich die Frequenz des Oszillators (1). Die Frequenzänderung wird mit Auswerteschaltung (14) bestimmt.

## Beschreibung

Zur Bestimmung des Okklusionskontakts zwischen zwei aneinander gepressten Körpern werden beispielsweise in der Zahnmedizin ab- oder verfärbende Folien verwendet, bei denen die Berührung zwischen Ober- und Unterkieferzähnen zu einer Verfärbung der Folie, aber auch zu einer Einfärbung der Kontaktpunkte an den Zähnen führt. Weiters kann die Folie eine Farbschicht mit einem in Kapseln eingeschlossenen Farbstoff aufweisen. Abhängig von der auf die Farbschicht wirkenden Druckkraft wird eine unterschiedliche Anzahl der Kapseln aufgebrochen und damit eine unterschiedliche Farbstoffmenge freigesetzt. Eine genaue Bestimmung der Stärke der Druckkraft, insbesondere des Okklusionsdrucks zwischen Zähnen, ist durch derartige Folien aber nicht möglich.

Zur Okklusionsdruckbestimmung sind deshalb auch elektrische Drucksensoren bekannt. So werden nach DE 31 17 284 A1, US 4,521,186 und EP 0 379 524 B1 Sensoren verwendet, die aus zwei jeweils mit Leiterbahnen versehenen Schichten bestehen, zwischen denen eine Widerstandsschicht vorgesehen ist. Bei Druckeinwirkung auf den Bissabschnitt des Sensors wird der Widerstand zwischen den Leiterbahnen der beiden Schichten geändert, und diese Änderung wird als Messgröße erfasst.

Die bekannten Sensoren sind in Aufbau und Handhabung relativ kompliziert, so dass sie sich gegenüber den herkömmlichen Okklusionskontaktfolien mit Verfärbung nicht durchsetzen konnten. Zudem weisen die bekannten Sensoren einen relativ dicken Bissabschnitt auf, worunter die Genauigkeit der Okklusionsmessung leidet. Zudem muß eine elektrische Kontaktierung der beiden Schichten mit den Leiterbahnen zur Zufuhr der Eingangs- und Ausgangssignale erfolgen. Da der Bissabschnitt aus hygienischen Gründen bei jedem Patienten gewechselt werden muß, ist das mit einem zusätzlichen Aufwand verbunden.

Im wesentlichen die gleichen Nachteile weisen Okklusionsdrucksensoren auf, die auf dem Piezoeffekt beruhen (z.B. US 4,592,727) . Zusätzlich haben die bekannten Piezosensoren den Nachteil, dass diese auf Grund des erforderlichen Verstärkers mit einem hochohmigen Eingangswiderstand in widrigen Umgebungsbedingungen (wie Wasser, Schmutz, Öl etc.) störanfällig sind.
Die DE 197 05 569 C1 wendet sich von den elektrischen Sensoren im Bissbereich ab und schlägt statt dessen eine Okklusionskraftübertragung mit einer Druckmittelflüssigkeit vor.

Aufgabe der Erfindung ist es, eine einfach aufgebaute Vorrichtung zur genauen Bestimmung des Drucks zwischen zwei aneinander gepressten Körpern, insbesondere des Dentalkontaktdrucks, bereitzustellen, die beispielsweise im Anwendungsbereich der Zahnmedizin ähnlich wie die bisherige Okklusionsfolie mit Verfärbung gehandhabt werden kann, und insbesondere keine elektrische Kontaktierung im Bissbereich erfordert.

Dies wird erfindungsgemäß mit der im Anspruch 1 gekennzeichneten Vorrichtung erreicht. In den Unteransprüchen 2 bis 31 sind vorteilhafte Ausge-staltungen der erfindungsgemäßen Vorrichtung wieder-gegeben. In den Ansprüchen 32 bis 37 sind bevorzugte Verwendungen der erfindungsgemäßen Vorrichtung sowie weitere vorteilhafte Ausgestaltungen für diese Verwendungen angegeben.

Nach der Erfindung ist zur Bestimmung des Drucks zwischen zwei aneinander gepressten Körpern ein Oszillator mit wenigstens einer Induktionsspule vorgesehen. Statt eines Oszillators können auch zwei oder mehrere Oszillatoren vorgesehen sein, beispielsweise ein Mess- und ein Referenzoszillator. Dabei kann die Differenz der Frequenz der beiden Oszillatoren hörbar z.B. nach dem sogenannten Schwebungssummer-Prinzip dargestellt werden.

Um die Induktionsspule wird ein wenigstens teilweise elektrisch leitfähiger Sensor angeordnet. Mit einer Auswerteschaltung wird die Frequenzänderung bei Änderung der Induktivität der Induktionsspule durch eine ohmsche und/oder kapazitive und/oder induktive Beeinflussung des Sensors bei Druckeinwirkung bestimmt.

Der Druck zwischen den aneinander gepressten Körpern kann erfindungsgemäß zahlenmäßig durch Maßeinheiten ermittelt werden oder z.B. auch als eine nicht auf Einheiten bezogene relative Messung. Durch die Bestimmung des Drucks ist auch eine Abstandsbestimmung zwischen den aneinander gespressten Körpern möglich.

Mit der erfindungsgemäßen Vorrichtung wird es insbesondere ermöglicht, den beim Zubeissen eines Ober- und eines Unterkiefers zwischen den Zähnen auftretenden Dentalkontaktdruck zu bestimmen. Durch diese Dentalkontaktdruckmessung wird auch die Bestimmung des Abstandes zwischen den aneinander gepressten Zähnen ermöglicht.

Durch die Erfindung wird aber nicht nur die Dentalkontaktdruckbestimmung bei einem Patienten, sondern auch an einem Zahnmodell, welches für die Anfertigung von Zahnersatz erforderlich sein kann, beispielsweise einem Meistermodell, welches sich vorzugsweise in einem Zahnartikulator befindet, ermöglicht.

Im Zusammenhang mit der Beschreibung der Dentalkontaktdruckbestimmung sind unter der Bezeichnung "Zähne" nicht nur die natürlichen Zähne, sondern auch jede Art von Zahnersatz, insbesondere restaurativer Zahnersatz wie beispielsweise Zahnfüllungen und Inlays, aber auch jeder abnehmbare oder festsitzende bzw. kombinierbare Zahnersatz wie beispielsweise Zahnprothesen, Zahnkronen, Zahnbrücken, Geschiebe oder Implantate und deren Aufbauten zu verstehen.

Unter der Dentalkontaktdruckbestimmung ist insbesondere die Okklusionsdruckbestimmung zwischen Ober- und Unterkieferzähnen zu verstehen, aber beispielsweise auch die Lateraldruckbestimmung zwischen zwei benachbarten Zähnen des Ober- oder Unterkiefers, beispielsweise zur Bestimmung des Interproximalabstandes.

Die erfindungsgemäße Vorrichtung ist zwar insbesondere zur Bestimmung des zwischen Zähnen auftretenden Kontaktdrucks bestimmt, sie kann jedoch generell zur Bestimmung des Drucks zwischen zwei aneinander gepressten Körpern verwendet werden.

Nachstehend wird die Erfindung aber in erster Linie im Zusammenhang mit der Dentalkontaktdruckbestimmung erläutert.

Der Oszillator der erfindungsgemäßen Vorrichtung weist eine bestimmte Eigenfrequenz auf, die beispielsweise im Bereich zwischen 10 kHz und 1 GHz liegen kann. Der Oszillator kann ein selbstschwingender Oszillator sein, oder durch einen Schwingungsgenerator angeregt werden.

Grundsätzlich kann jeder bekannte LC-Oszillator, wie zum Beispiel Meißner-, Hartley- oder Colpitts-Oszillator, verwendet werden, auch kommen RL- und Quarzgeneratoren in Betracht. Vorzugsweise werden aber Oszillatoren verwendet, die mit Invertern, insbesondere Schmitt-Trigger-Invertern, NAND- und/oder NOR-Gattern versehen sind. Diese Oszillatoren sind deswegen interessant, weil sie einen sehr einfachen und kostengünstigen Aufbau aufweisen. Als besonders geeignet hat sich eine Schaltung herausgestellt, die eine frequenzbestimmende Induktivität als Transkonduktanz zwischen Eingang und Ausgang bei der Inverterschaltung verwendet.

Unter Sensor im Zusammenhang mit der erfindungsgemäßen Vorrichtung ist nicht nur der druckaufnehmende Bereich, sondern auch jede Wicklung, welche um die Induktionsspule des Oszillators anordenbar ist, sowie erforderliche Verbindungen, insbesondere elektrische Zuleitungen zwischen druckaufnehmendem Bereich und Wicklung, zu verstehen.

Der Sensor kann beispielsweise durch eine elektrisch leitfähige Folie oder einen elektrisch leitfähigen Draht gebildet werden. Weiters kann der Sensor beispielsweise aus einem Piezoplättchen, bei welchem die elektrischen Zuleitungen um die Induktionsspule anordenbar sind, gebildet werden.

Die elektrisch leitfähigen Zuleitungen, insbesondere die elektrisch leitfähige Folie oder der elektrisch leitfähige Draht werden wenigstens einmal um die Induktionsspule des Oszillators gewickelt. Wenn sie mehrmals um die Induktionsspule geschlungen werden, wird der Messeffekt verstärkt.

Der Sensor kann auch durch ein Material mit elektrisch leitfähigen Eigenschaften gebildet werden, welches zumindest im druckaufnehmenden Bereich der aneinander gepressten Körper mit einer elektrisch isolierenden Schicht versehen ist. Diese elektrisch isolierende Schicht kann beispielsweise durch einen elektrisch isolierenden Kunststoff oder beispielsweise auch durch ein Metalloxid gebildet werden. Diese elektrisch isolierende Schicht kann bei Druckeinwirkung der aneinander gepressten Körper im Bereich der Berührungsflächen zerstört werden, sodass dadurch der Übergangswiderstand beeinflusst wird. Dadurch kann die erfindungsgemäße Vorrichtung auf die in den verschiedensten Anwendungsbereichen erforderlichen Messbereiche durch die Auswahl der Dicke der elektrisch isolierenden Schicht des Sensors abgestimmt werden.

Der Sensor braucht jedoch grundsätzlich keine durch Druckeinwirkung zerstörbare, elektrisch isolierende Schicht aufzuweisen. Vielmehr ist es möglich, einen Sensor aus elektrisch leitfähigem Material einzusetzen, dessen Übergangswiderstand sich beim Zusammenpressen zwischen den beiden Körpern bzw. Zähnen ändert, beispielsweise in der Größenordnung von 0,1 mΩ bis 100 mΩ, um eine Frequenzverschiebung des Oszillators herbeizuführen.

Wenn der Dentalkontaktdruck bestimmt wird, kann die elektrisch leitfähige Folie beispielsweise als Messstreifen ausgebildet sein. Weiters kann der Messstreifen durch eine Folie aus einem elektrisch leitfähigen Material gebildet und, beispielsweise seiner Innenseite, mit einer elektrisch isolierenden Schicht versehen sein. Das elektrisch leitfähige Material ist insbesondere ein metallischer Werkstoff, beispielsweise ein Aluminium- oder Kupferwerkstoff, also Aluminium oder eine Aluminiumlegierung bzw. Kupfer oder eine Kupferlegierung. Statt einer Metallfolie kann jedoch auch beispielsweise eine elektrisch leitende Kunststofffolie verwendet werden. Die elektrisch isolierende Schicht des Messstreifens kann beispielsweise ein Metalloxid oder beispielsweise auch eine Piezoschicht sein. Es versteht sich, dass bei Vorhandensein einer elektrisch isolierenden Schicht diese nur in jenem Bereich des Messstreifens vorgesehen sein muss, der zwischen den aneinander gepressten Körpern bzw. Zähnen liegt. Auch kann der Messstreifen an der Aussenseite mit einer elektrisch isolierenden Schicht versehen sein.

Der Sensor kann weiters aus einem elektrisch leitfähigem Material und einem elektrisch isolierenden Material mit ferroelektrischen Eigenschaften, beispielsweise aus einer elektrisch isolierenden Schicht mit ferroelektrischen Eigenschaften, gebildet werden. Vorzugsweise werden hierfür piezoelektrische Materialien verwendet. Durch die hohe Dielektrizitätskonstante dieser Materialien entsteht beim Sensor eine zusätzliche kapazitive Belastung, die zu einer Frequenzänderung des Oszillators führt. Die Dielektrizitätskonstante wird durch Veränderung des Drucks zwischen den aneinander gepressten Körpern beeinflusst.

Ein weiterer Vorteil der erfindungsgemäßen Vorrichtung besteht darin, dass bei Verwendung eines Piezoelementes als Teil des Sensors hierfür kein Verstärker benötigt wird, da dieses als kapazitive Last niederohmig an die Sensorelektronik angekoppelt wird. Dadurch wird die Bestimmung des Drucks zwischen zwei aneinander gepressten Körpern auch bei widrigen Umgebungsbedingungen, insbesondere in der feuchten Umgebung der Zahnmedizin, ermöglicht.

Der Sensor, insbesondere die Folie bzw. der Messtreifen, kann auch aus einem ferromagnetischen Material, insbesondere einem ferromagnetischen Material mit magnetoelastischen Eigenschaften gebildet werden. So haben sich amorphe Legierungen, sogenanntes metallisches Glas, als geeignet erwiesen. Materialien mit magnetoelastischen Eigenschaften zeichnen sich dadurch aus, dass sich ihre Permeabilität bei mechanischer Belastung ändert. Durch Verwendung von Materialien mit ferromagnetischen Eigenschaften wird eine Verbesserung des Messeffektes durch eine stärkere Beeinflussung der Induktivität ermöglicht.

Der vorzugsweise als Messstreifen ausgebildete Sensor kann aus mehreren Schichten bestehen, die abwechselnd aus einem elektrisch leitfähigen und einem elektrisch isolierenden Material bestehen. In einem solchen Messstreifen bilden die Schichten aus elektrisch leitfähigem Material mehrere Wicklungen, die parallel zum Ablauf der Druckeinwirkung der aneinander gepressten Körper nacheinander aktiviert werden können. Dadurch werden durch die Anzahl bzw. der Auswahl der Schichtmaterlien verschiedene Messund Proportionalbereiche ermöglicht.

Der Sensor kann zur Befestigung am Gehäuse des Oszillators beispielsweise mit einer selbsthaftenden Schicht versehen sein. Auch ist es möglich, den Messstreifen mit einer Federklammer zu versehen, um ihn am Gehäuse zu befestigen. Auch ist es möglich, das Gehäuse des Oszillators so auszuführen, dass der Sensor mechanisch, beispielsweise mit einer offenen Hülse oder einer Federklammer ähnlich einem Kugelschreiber, darauf befestigt werden kann.

Es gibt weitere zahlreiche Möglichkeiten, um den Sensor und das Gehäuse lösbar miteinander zu verbinden. So können an oder im Gehäuse Öffnungen vorgesehen sein, durch die der Sensor, welcher beispielsweise als Messstreifen ausgebildet ist, gesteckt oder eingefädelt wird.

Der Sensor kann auch auf ein elektrisch isolierendes Material aufgebracht sein. Das Material kann z.B. Papier oder ein Kunststoff, insbesondere ein elastischer Kunststoff, sein. Der Kunststoff ist vorzugsweise so beschaffen, dass der Sensor durch Sterilisation gereinigt werden kann.

Der Sensor wird beispielsweise durch eine Metallfolie gebildet. Als Metall kommen z.B Edelmetalle, wie Gold oder Silber, in Frage, die auch auf ein isolierendes Trägermaterial z.B. durch Aufdampfen aufgebracht sein können.

Als besonders vorteilhaft hat sich ein Messstreifen aus einem Aluminiumwerkstoff herausgestellt, der an der Innenseite mit einer Aluminiumoxidschicht passiviert ist. Die Dicke der Aluminiumoxidschicht kann durch Eloxieren des Aluminiumstreifens optimiert werden. Der Messstreifen weist dabei eine Dicke von vorzugsweise weniger als 200 *µ*m, insbesondere weniger als 100 *µ*m auf. Wenn auf den Messstreifen gebissen wird, kann an den Okklusionsstellen die elektrisch isolierende Schicht zerstört werden. Dadurch entsteht, wenn der Messstreifen einmal um die Induktionsspule gewickelt worden ist, um die Induktionsspule eine weitere Wicklung, die den Oszillator verstimmt. Diese Wicklung kann die Induktivität des Oszillators beispielsweise in einer Transformator-schaltung belasten. Die durch die Verstimmung hervor-gerufene Frequenzänderung kann sowohl eine Erhöhung als auch eine Verringerung der Eigenfrequenz des Oszillators hervorrufen, je nach den elektromagnetischen Verhältnis-sen im frequenzbestimmenden Schwingkreis und der Wahl des Arbeitspunktes.

Statt oder zusätzlich zu der so hervorgerufenen induktiven Belastung kann die Frequenzänderung auch durch eine kapazitive und/oder ohmsche Belastung hervorgerufen werden. So weist die durch Zerstörung der isolierenden Schicht gebildete Wicklung einen Ohmschen Widerstand auf, der unter anderem von der mechanischen Kontaktierung an den Okklusionsstellen abhängt.

Weiterhin liegen bei dem um die Induktionsspule gewickelten Messstreifen die beiden Endabschnitte des Messstreifens, auf die der zu bestimmende Druck einwirkt, also auf die gebissen wird, mit ihren Innenseiten mit der elektrisch isolierenden oder dielektrischen Schicht aufeinander. Damit bildet der Messstreifen zugleich eine Kapazität, die zu einer Frequenzänderung führt, je nach dem, wie sich beim Biss die Dicke der dielektrischen Schicht und damit der Abstand der elektrisch leitfähigen Folienendabschnitte des Streifens und die Dielektrizitätskonstante ändern.

Die Auswerteschaltung der erfindungsgemäßen Vorrichtung kann eine Phase-Lock-Loop-(PLL)-Schaltung sein und/oder beispielsweise ein PC, dem die Ausgangssignale des Oszillators bzw. der PLL-Schaltung zugeführt werden.

Der Oszillator kann in einem Gehäuse mit einem hülsenförmigen Abschnitt angeordnet sein, um den der Messstreifen gewickelt wird. Vorzugsweise ist der Oszillator drahtlos mit der Auswerteschaltung verbunden. Zur Messung des Dentalkontaktdrucks braucht damit nur der Messstreifen um das hülsenförmige, beispielsweise bleistiftgroße Gehäuse, einmal geschlungen zu werden, worauf der Patient auf die aufeinanderliegenden Endabschnitte des um das Gehäuse des Oszillators gewickelten Messstreifens beißt. Selbstverständlich kann der Sensor bzw. die Folie oder der Messstreifen mit dem Oszillator und/oder Auswerteschaltung auch über ein Kabel verbunden sein.

Der erfindungsgemäße Messstreifen weist vorzugsweise eine einer herkömmlichen Okklusionsfolie mit Verfärbung entsprechende Dicke und Flexibilität auf. Er kann an seiner Außenseite zusätzlich mit einer Beschichtung zur Erfassung der Okklusionspunkte durch Abfärbung oder Verfärbung versehen werden, um weitere Messdaten zu gewinnen.

Die Ausgangssignale der Auswerteschaltung können beispielsweise in einem Speicher abgelegt und/oder einer Anzeige zugeführt werden. Die Anzeige kann beispielsweise durch einen Balken aus Leuchtdioden gebildet sein.

Da die erfindungsgemäße Vorrichtung im niederohmigen Bereich arbeitet, wird sie durch galvanische Effekte, die z.B. im Speichel auftreten können, nicht beeinflusst.

Nachstehend ist die Erfindung an Hand der Zeichnung beispielhaft näher erläutert. Darin zeigt:
- Figur 1: eine Ausführungsform der Schaltung der erfindungsgemäßen Vorrichtung;
- Figur 2: verschiedene Kennlinien der Frequenzänderung bei auf den Messstreifen/Sensor einwirkender Kraft;
- Figur 3: einen um das Gehäuse des Oszillators gewickelten Messstreifen in perspektivischer Wiedergabe;
- Figur 4: schematisch den Okklusionsdruck vor und nach einer Dentalfüllung; und
- Figur 5: eine um das Gehäuse des Oszillators gewickelte sekundäre Messwicklung mit einem Piezoelement in perspektivischer Wiedergabe.

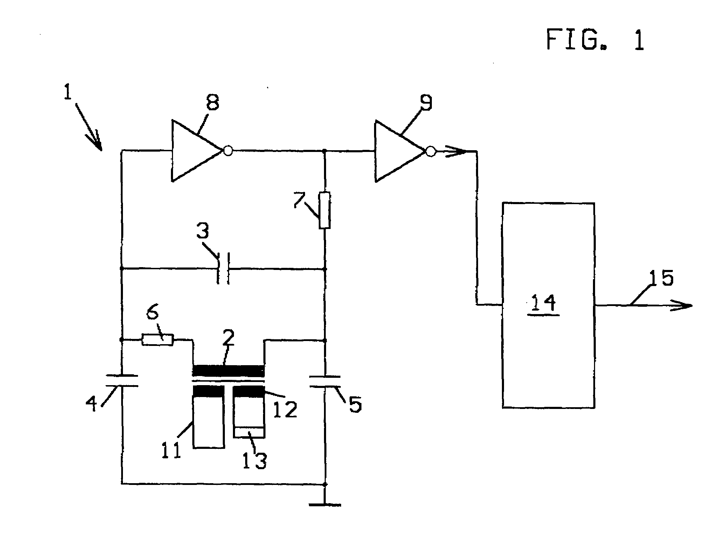

Gemäß Figur 1 weist der Oszillator 1 einen selbstschwingenden Schwingkreis mit einer Induktionsspule 2, mehreren Kapazitäten 3, 4, 5, zwei ohmschen Widerständen 6, 7 und zwei Schmitt-Trigger-Invertern 8, 9 zur Bildung der Ausgangssignale auf. Die beiden Schmitt-Trigger-Inverter 8, 9 werden mit einer nicht dargestellten, zum Beispiel 5 V-Kleinspannungsquelle (z.B. Akku oder Batterie) versorgt.

Der Sensor 11 ist als Messstreifen ausgebildet und, wie in Figur 1 schematisch dargestellt, in Transformatorschaltung zur Induktionsspule 2 angeordnet. Ferner ist eine Sekundärwicklung 12 mit einem ohmschen Widerstand 13 oder einer anderen Last in Transformatorschaltung zu der Induktionsspule 2 angeordnet. Mit der Sekundärwicklung 12 kann die Kennlinie der Frequenzänderung bei Einwirkung einer Kraft auf den Sensor 11 optimiert werden.

Die Induktionsspule 2 kann auch als Differentialtrafo oder Differentialspule ausgebildet sein. Diese Induktionsspulenausführung ist insbesondere dann vorteilhaft, wenn, wie vorstehend erwähnt, zwei oder mehrere Oszillatoren vorgesehen sind. Eine Differentialspule liegt dann vor, wenn in Fig. 1 die Wicklung 12 wegfällt. Hingegen ist die Wicklung 12 beim Differentialtrafo vorhanden und symmetrisch zur Primärwicklung 2 angeordnet.

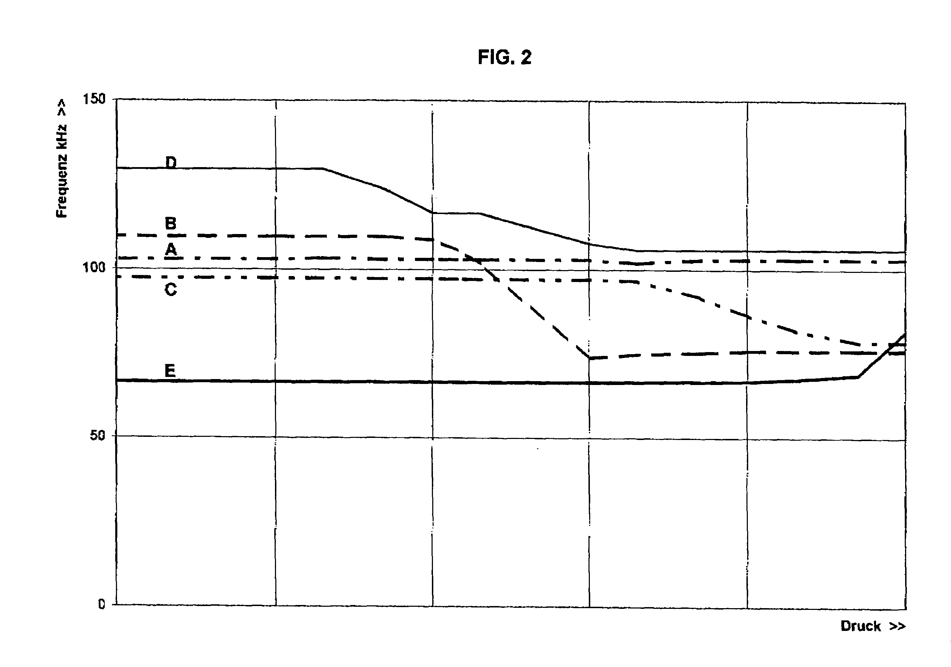

In Figur 2 sind fünf unterschiedliche Messkennlinien A, B, C, D und E dargestellt, wobei die Kennlinie A weitgehend unbrauchbar ist, während die Kennlinie B einen langen Proportionalbereich, die Kennlinie C einen kürzeren Proportionalbereich und die Kennlinie D zwei proportionale Bereiche aufweist. Die Kennlinie E zeigt eine Erhöhung der Oszillatorfrequenz bei Steigerung der Messkraft.

Die Ausgangssignale des Inverters 9 werden der Auswerteschaltung 14 zugeführt, die beispielsweise durch eine PLL-Schaltung gebildet sein kann, d.h. eine Schaltung, die die Eingangsfrequenz mit einer internen Frequenz vergleicht, und bei einer Frequenzdifferenz ein Ausgangssignal 15 erzeugt. Während von dem Schmitt-Trigger-Inverter 9 zur Signalauskopplung ein digitales Ausgangssignal erzeugt wird, wird durch die PLL-Schaltung ein analoges Ausgangssignal gebildet. Dieses kann mit einem nicht dargestellten Analog-Digital-Konverter in ein digitales Signal umgewandelt und in einem nicht dargestellten Speicherbaustein abgelegt und auf einem gleichfalls nicht dargestellten Leuchtdioden-Balken angezeigt werden. So können beispielsweise drei bis zehn Speicherbausteine vorhanden sein, so dass nacheinander drei bis zehn Messungen auf drei bis zehn Leuchtbalken angezeigt werden können. Diese Darstellung gestattet es dem Zahnarzt, die Bisskraft und den Zahnabstand vor, während und nach den durchzuführenden dentalen Arbeiten mehrfach zu messen.

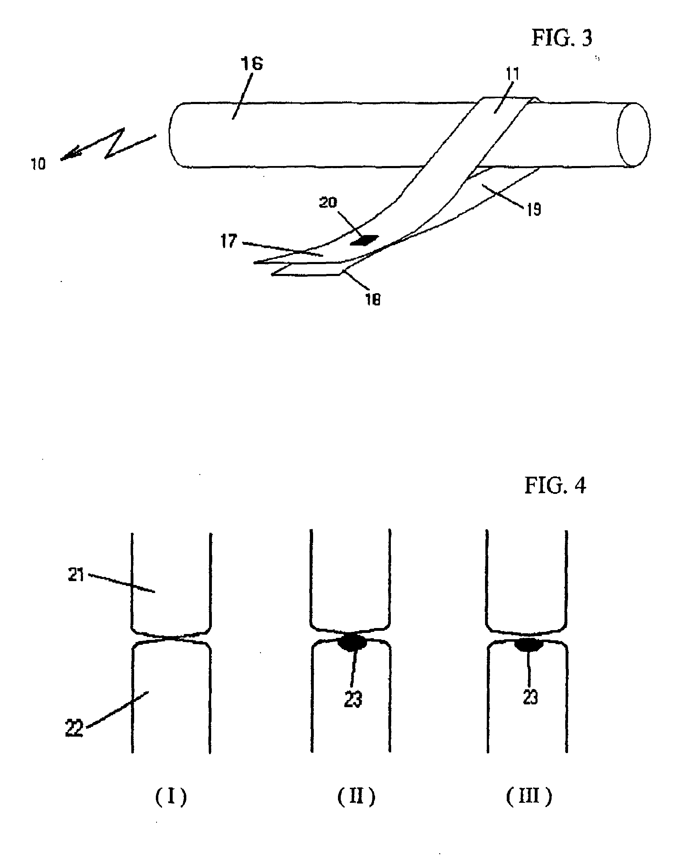

Gemäß Figur 3 ist der Oszillator 1 (Fig. 1) in einem hülsenförmigen, etwa bleistiftgroßen Gehäuse 16 angeordnet. Der Oszillator ist, wie durch den Pfeil 10 angedeutet, drahtlos mit der Auswerteschaltung 14 (Fig. 1) verbunden.

Der Messstreifen 11 ist in Figur 3 einmal um das Gehäuse 16 im Bereich der Induktionsspule 2 (Fig. 1) herumgewickelt. Seine Endabschnitte 17, 18 bilden damit aufeinanderliegende Fahnen. Der Messstreifen 11, der eine etwa einem Zahn entsprechende Breite aufweist, besteht beispielsweise aus einer Aluminiumfolie, die an ihrer Innenseite 19 mit einer isolierenden Aluminiumoxidschicht versehen ist. Zur Messung des Okklusionsdrucks wird auf die aufeinanderliegenden Fahnen 17, 18 gebissen. Dadurch wird an der Okklusionsstelle 20 die Aluminiumoxidschicht zerstört, oder ihre Schichtdicke zumindest derart verringert, dass eine geschlossene, elektrisch leitende Windung entsteht, die die Induktivität und damit die Frequenz des Oszillators 1 (Fig. 1) ändert. Um den Messeffekt zu verstärken, kann der Messstreifen 11 auch mehrmals um das Gehäuse gewickelt sein. Auch kann der Messstreifen aus einer Metallfolie ohne isolierende Schicht bestehen. Der Übergangswiderstand zwischen den Fahnen 17, 18 hängt dann vom Okklusionsdruck ab.

Wenn gemäß Figur 4 der Patient beispielsweise eine Füllung in dem mit dem Oberkieferzahn 21 okkludierenden Unterkieferzahn 22 erhält, wird der Okklusionsdruck gemäß (I) vor der Füllung als Referenz gemessen und abgespeichert. Dann wird der Zahn 22 gemäß (II) mit der Füllung 23 versehen und die Füllung so lange abgeschliffen (III), bis sich der Referenz-Okklusionsdruck wie vor der Füllung (I) einstellt. Beim Einsetzen von Zahnersatz wie beispielsweise Kronen, Brücken usw. hat es sich als vorteilhaft erwiesen, wenn der Druck bzw. der Abstand der benachbarten Zähne des Zahnersatzes vor, während und nach dem Einsetzen in einer Referenzmessung bestimmt wird.

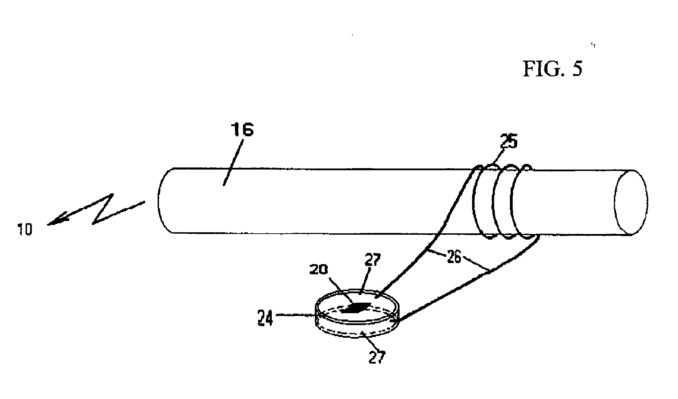

In Figur 5 ist eine sekundäre Messwicklung 25 mehrmals um das Gehäuse 16 im Bereich der Induktionsspule 2 (Fig. 1) herumgewickelt. Die beiden Zuleitungen 26 der Messwicklung 25 sind mit den beiden Piezoelektroden 27 des Piezoelementes 24 verbunden. Durch Druckausübung auf die Okklussionsstelle 20 wird das Piezoelement 24 wie vorher beschrieben beeinflusst, dadurch der Oszillator verstimmt und so die Bestimmung des Okklusionsdruckes ermöglicht.

## Patentansprüche

1. Vorrichtung zur Bestimmung des Drucks zwischen zwei aneinander gepressten Körpern, **gekennzeichnet durch** wenigstens einen Oszillator (1) mit wenigstens einer Induktionsspule (2), einen Sensor (11) aus einem wenigstens teilweise elektrisch leitfähigem Material, welcher um die Induktionsspule (2) anordenbar ist, und eine Auswerteschaltung (14) zur Bestimmung der Frequenzänderung bei Änderung der Induktivität der Induktionsspule (2) **durch** Druckeinwirkung auf den Sensor (11).

2. Vorrichtung nach Anspruch 1, **dadurch gekennzeichnet, dass** der Oszillator (1) neben wenigstens einer Induktionsspule (2) wenigstens eine Kapazität (3, 4, 5) und/oder wenigstens einen Widerstand (6, 7) aufweist.

3. Vorrichtung nach einem der vorstehenden Ansprüche, **dadurch gekennzeichnet, dass** der Sensor (11) ferromagnetische Eigenschaften aufweist.

4. Vorrichtung nach Anspruch 3, **dadurch gekennzeichnet, dass** der ferromagnetische Sensor (11) magnetoelastische Eigenschaften aufweist.

5. Vorrichtung nach einem der vorstehenden Ansprüche, **dadurch gekennzeichnet, dass** der Oszillator (1) in einem Gehäuse (16) vorgesehen ist, das einen Abschnitt mit der Induktionsspule (2) aufweist, um den der Sensor (11) anordenbar ist.

6. Vorrichtung nach einem der vorstehenden Ansprüche, **dadurch gekennzeichnet, dass** der Sensor (11) und/oder das Gehäuse (16) aneinander befestigbar ausgebildet sind.

7. Vorrichtung nach Anspruch 6, **dadurch gekennzeichnet, dass** zur Befestigung des Sensors (11) am Gehäuse (16) der Sensor (11) und/oder das Gehäuse (16) mit einer Haftschicht und/oder einer Federklammer versehen sind.

8. Vorrichtung nach einem der vorstehenden Ansprüche, **dadurch gekennzeichnet, dass** der druckaufnehmende Bereich des Sensors (11) und/oder die um die Induktionsspule (2) anordenbare Wicklung aneinander befestigbar ausgebildet sind.

9. Vorrichtung nach einem der vorstehenden Ansprüche, **dadurch gekennzeichnet, dass** der Sensor (11) wenigstens im druckaufnehmenden Bereich mit einer elektrisch isolierenden Schicht versehen ist.

10. Vorrichtung nach einem der vorstehenden Ansprüche, **dadurch gekennzeichnet, dass** der Sensor (11) wenigstens teilweise durch eine elektrisch leitende Folie und/oder wenigstens teilweise durch einen elektrisch leitenden Draht gebildet wird, die bzw. der wenigstens einmal um die Induktionsspule (2) wickelbar ist.

11. Vorrichtung nach einem der vorstehenden Ansprüche, **dadurch gekennzeichnet, dass** der Sensor (11) wenigstens teilweise auf ein elektrisch isolierendes Material aufgebracht ist.

12. Vorrichtung nach Anspruch 11, **dadurch gekennzeichnet, dass** das elektrisch isolierende Material ein Kunststoff ist.

13. Vorrichtung nach einem der vorstehenden Ansprüche, **dadurch gekennzeichnet, dass** der Sensor (11) aus wenigstens einer Folie aus mehreren Schichten besteht, die abwechselnd aus einem elektrisch leitfähigen und einem elektrisch isolierenden Material bestehen.

14. Vorrichtung nach einem der vorstehenden Ansprüche, **dadurch gekennzeichnet, dass** die elektrisch leitende Folie und/oder der elektrisch leitende Draht eine elektrisch isolierende Schicht aufweisen.

15. Vorrichtung nach Anspruch 9 oder 14, dadurch gekennnzeichnet, dass die elektrisch isolierende Schicht ferroelektrische Eigenschaften aufweist.

16. Vorrichtung nach einem der vorstehenden Ansprüche, **dadurch gekennzeichnet, dass** die elektrisch isolierende Schicht piezoelektrische Eigenschaften aufweist.

17. Vorrichtung nach einem der vorstehenden Ansprüche, **dadurch gekennzeichnet, dass** die elektrisch isolierende Schicht durch Druckeinwirkung zerstörbar ausgebildet ist.

18. Vorrichtung nach einem der vorstehenden Ansprüche, **dadurch gekennzeichnet, dass** die elektrisch leitende Folie eine Metallfolie ist.

19. Vorrichtung nach Anspruch 18, **dadurch gekennzeichnet, dass** die Metallfolie eine Aluminiumfolie ist.

20. Vorrichtung nach Anspruch 19, **dadurch gekennzeichnet, dass** die Aluminiumfolie an ihrer Innenseite (19) eine Aluminiumoxidschicht als elektrisch isolierende Schicht aufweist.

21. Vorrichtung nach einem der vorstehenden Ansprüche, **dadurch gekennzeichnet, dass** die elektrisch leitende Folie als Messstreifen ausgebildet ist.

22. Vorrichtung nach einem der vorstehenden Ansprüche, **dadurch gekennzeichnet, dass** der Sensor aus wenigstens einem Piezoelement (24) gebildet wird, das mit der sekundären Messwicklung (25) im Bereich der Induktionsspule (2) verbunden ist.

23. Vorrichtung nach Anspruch 22, **dadurch gekennzeichnet, dass** das Piezoelement im druckaufnehmenden Bereich eine Dicke von höchstens 150 *µ*m, vorzugsweise höchstens 100 *µ*m aufweist.

24. Vorrichtung nach Anspruch 1, **dadurch gekennzeichnet, dass** der Induktionsspule (2) eine Sekundärwicklung (12) mit einer Last (13) zur Optimierung der Messkennlinie (A, B, C, D, E) zugeordnet ist.

25. Vorrichtung nach einem der vorstehenden Ansprüche, **dadurch gekennzeichnet, dass** die Induktionsspule als Differentialtransformator und/oder Differentialspule ausgebildet ist.

26. Vorrichtung nach Anspruch 1, **dadurch gekennzeichnet, dass** die Auswerteschaltung (14) eine Phase-Lock-Loop-Schaltung ist.

27. Vorrichtung nach Anspruch 1, **dadurch gekennzeichnet, dass** das Ausgangssignal des Oszillators (1) einem PC oder einem Mikrocomputer zugeführt wird.

28. Vorrichtung nach Anspruch 1, **dadurch gekennzeichnet, dass** der Oszillator (1) drahtlos mit der Auswerteschaltung (14) verbunden ist.

29. Vorrichtung nach Anspruch 1, **dadurch gekennzeichnet, dass** das Ausgangssignal (15) der Auswerteschaltung (14) einer Anzeige zugeführt wird.

30. Vorrichtung nach Anspruch 29, **dadurch gekennzeichnet, dass** die Anzeige durch Leuchtdioden gebildet wird.

31. Vorrichtung nach einem der vorstehenden Ansprüche, **dadurch gekennzeichnet, dass** ein Speicher zum Ablegen der Ausgangssignale (15) der Auswerteschaltung (14) vorgesehen ist.

32. Verwendung der Vorrichtung nach einem der vorstehenden Ansprüche zur Bestimmung des Dentalkontaktdrucks.

33. Verwendung nach Anspruch 32, wobei als Dentalkontaktdruck der Okklusionsdruck zwischen Zähnen bestimmt wird.

34. Verwendung nach Anspruch 32 oder 33, wobei ein Sensor (11) mit einer dem jeweiligen Zahn entsprechenden Breite verwendet wird.

35. Verwendung nach einem der Ansprüche 32 bis 34, wobei der Sensor im druckaufnehmenden Bereich eine Dicke von höchstens 100 *µ*m, vorzugsweise höchstens 70 *µ*m aufweist.

36. Verwendung nach einem der Ansprüche 32 bis 35, wobei ein Sensor verwendet wird, der im Bereich der Druckflächen der aneinander gepressten Zähne mit einer abfärbenden und/oder verfärbenden Beschichtung zur Erfassung der Okklusionskontaktpunkte versehen ist.

37. Verwendung nach einem der Ansprüche 32 bis 36, wobei ein Sensor verwendet wird, bei welchem die elektrisch isolierende Schicht im Bereich der Druckflächen der aneinander gepressten Zähne aus der abfärbenden und/oder verfärbenden Beschichtung zur Erfassung der Okklusionskontaktpunkte gebildet wird.
